(19) **Europäisches Patentamt**

**European Patent Office**

**Office européen des brevets**

(11) **EP 1 354 564 B1**

(12) **EUROPÄISCHE PATENTSCHRIFT**

(45) Veröffentlichungstag und Bekanntmachung des Hinweises auf die Patenterteilung:
**15.09.2004 Patentblatt 2004/38**

(51) Int Cl.$^7$: **A61B 19/00**

(21) Anmeldenummer: **02008185.7**

(22) Anmeldetag: **16.04.2002**

(54) **Marker für ein Instrument und Verfahren zur Lokalisation eines Markers**

Instrument marker and method for localizing a marker

Marqueur pour un instrument et procédé de localisation d'un marqueur

(84) Benannte Vertragsstaaten:
**AT BE CH CY DE DK ES FI FR GB GR IE IT LI LU MC NL PT SE TR**

(43) Veröffentlichungstag der Anmeldung:
**22.10.2003 Patentblatt 2003/43**

(73) Patentinhaber: **BrainLAB AG**
**85551 Kirchheim/Heimstetten (DE)**

(72) Erfinder: **Vilsmeier, Stefan**
**6330 Kufstein (AT)**

(74) Vertreter: **Schwabe - Sandmair - Marx**
**Stuntzstrasse 16**
**81677 München (DE)**

(56) Entgegenhaltungen:
| | |
|---|---|
| WO-A-01/30254 | WO-A-97/29710 |
| US-A- 5 158 084 | US-A- 5 485 667 |
| US-A- 5 489 457 | US-A- 5 600 330 |
| US-A- 5 662 111 | US-A- 5 729 129 |

## EP 1 354 564 B1

**Beschreibung**

Die vorliegende Erfindung bezieht sich auf einen Marker für ein Instrument, bevorzugt für ein rotationssymmetrisches medizinisches Instrument, auf ein System, welches solche Marker verwendet, sowie auf Verfahren zur Lokalisation mindestens eines Markers. Ein erfindungsgemäßer Marker kann zum Lokalisieren und/oder Navigieren eines Instrumentes, insbesondere bei "Image Guided Surgery" verwendet werden. Allgemein kann die Erfindung in der Mikrochirurgie, Orthopädie, spinalen oder cranialen Chirurgie oder in anderen Bereichen eingesetzt werden.

Zur Navigation oder Lokalisation von Instrumenten, wie zum Beispiel medizinischen Instrumenten, ist es bekannt einen sogenannten Referenzstern an dem medizinischen Instrument anzubringen, an dessen Enden Marker, zum Beispiel aktiv lichtemittierende Elemente oder passive reflektierende Oberflächen angeordnet sind. Eine Kamera, welche das von den Markern emittierte oder reflektiere Licht erfasst, kann Informationen liefern, aus welcher die Lage der Marker im Raum und damit die Lage des mit den Markern bzw. dem Referenzstern verbundenen Instruments ermittelt werden kann. Damit kann ein Instrument navigiert werden, d.h. aufgrund der so ermittelten räumlichen Lage des Instrumentes kann das Instrument computerunterstützt präzise an einen gewünschten Ort gebracht werden, z.B. an eine bestimmte Stelle eines Körpers, welcher bevorzugt ebenfalls mit Markern verbunden ist.

Weist das Instrument zum Beispiel eine Achse auf, welche während des Einsatzes des Instruments gedreht wird, wie zum Beispiel ein Schraubenzieher oder ein Bohrer, so ist es nicht vorteilhaft, Marker einfach an dieser Achse anzubringen, da diese aufgrund einer Drehung aus dem Sichtbereich einer Kamera herausbewegt werden. Zur Lösung dieses Problems wurde bereits vorgeschlagen, an dem sich drehenden Instrument ein Lager anzubringen, an welchem Marker befestigt werden können. Jedoch muss eine Bedienperson bei der Verwendung des drehbaren Instruments stets darauf achten, dass die an dem Lager angebrachten Marker in Richtung der Kameras gehalten werden, was die Handhabung des Instruments erschwert.

Weiterhin bedeutet ein Lager für ein Instrument einen zusätzlichen konstruktiven Aufwand, der sich in höheren Herstellungskosten und größerer Störanfälligkeit niederschlägt. Ebenso ist ein relativ großer Aufwand erforderlich, um eine solche konstruktiv aufwändig gestaltete Vorrichtung zu sterilisieren.

Aus der DE 196 39 615 A1, DE 195 36 180 A1, DE 100 00 937 A1 und DE 296 23 941 U1 der Anmelderin sind Verfahren und Vorrichtungen zum Lokalisieren und Navigieren von Instrumenten bekannt.

US 5,485,667 offenbart ein Verfahren zum Anbringen eines Markers an einem medizinischen Instrument, um das distale Ende des medizinischen Instruments genau zu positionieren, wobei der Marker bei einer Röntgenaufnahme sichtbar ist.

WO 01/30254 A1 offenbart einen Katheter mit einer Mehrzahl von für Röntgenstrahlen undurchlässigen Markern, welche entlang des Katheters angeordnet sind und keine Spiegelsymmetrien außer bezüglich Ebenen orthogonal einer Längsachse des Katheters aufweisen, wobei eine Ausrichtung des Katheters relativ zu einer Ebene unter Verwendung einer Projektion eines Bildes des Markers auf die Ebene bestimmt wird.

US 5,662,111 offenbart ein System und ein Verfahren zur quantitativen Bestimmung der Position der Anatomie eines Patienten und der Position eines Instruments mit zwei Markern in Bezug auf die anatomischen Daten, welche durch Computertomographie - oder Kernspinresonanz-Verfahren gewonnen wurden.

Es ist eine Aufgabe der vorliegenden Erfindung, einen Marker für ein Instrument, ein System, welches solche Marker verwendet, sowie Verfahren zur Lokalisation mindestens eines Markers vorzuschlagen, mit welchen Instrumente, wie zum Beispiel rotationssymmetrische Instrumente, einfach lokalisiert werden können.

Diese Aufgabe wird durch die in den unabhängigen Ansprüchen definierte Erfindung gelöst. Vorteilhafte Ausführungsformen ergeben sich aus den abhängigen Ansprüchen.

Ein Marker, welcher bevorzugt für bei der Verwendung zu bewegende oder zu drehende Instrumente, insbesondere für Instrumente mit einer Drehachse oder Instrumente mit einem rotationssymmetrischen Bereich verwendet wird, ist so ausgebildet, dass er an mindestens einem Bereich des Instruments entlang mindestens eines Teils eines um das Instrument umlaufenden Oberflächenbereichs angebracht werden kann. Der Marker kann zum Beispiel ein um ein Instrument, zum Beispiel ein zylinderförmiges Objekt an der Oberfläche mit beispielsweise in etwa konstanter Breite umlaufendes Band, mehrere Bandabschnitte oder ein umlaufender kontinuierlicher Ring oder einzelne Ringabschnitte sein, so dass das zylinderförmige Objekt um die Längsachse gedreht werden kann, wobei unabhängig vom Drehwinkel des zylinderförmigen Objekts immer mindestens ein Teilbereich des umlaufenden Ringes oder Bandes von einer Kamera erfasst werden kann, sofern die Kamera nicht auf einer Richtung in Verlängerung der Längsachse des zylinderförmigen Objekts positioniert ist, sondern einen seitlichen Bereich des zylinderförmigen Objekts erfassen kann.

Der Marker kann kontinuierlich aus einem Element, oder alternativ auch aus mehreren Einzelelementen bestehen, welche um die Oberfläche eines Instruments herum so verteilt angeordnet werden können, dass unabhängig von der Drehrichtung des Instruments mindestens ein Teil des Markers oder ein Einzelelement von einer seitlich zum Instrument angeordneten Kamera erfasst werden kann. Beispielsweise kann ein Ring verwendet werden, welcher an bestimmten Teilen seines Umfangs unterbrochen ist oder es können verschiedene einzelne lichtemittierende oder lichtreflektierende Oberflächen mit in etwa gleicher oder auch verschiedener Geometrie um die Oberfläche des Instruments herum so angebracht werden, dass unabhängig von der Drehrichtung des Instruments mindestens ein Markerelement bei einer seitlichen Betrachtung des Instruments gesehen werden kann.

Allgemein soll ein Marker, welcher aus einem zusammenhängenden oder mehreren einzelnen diskreten Elementen bestehen kann, so um mindestens einen Teilbereich der Oberfläche eines Instruments verteilt angeordnet werden können, dass unabhängig von der Ausrichtung oder Drehung des Instruments mindestens ein Teilbereich des Markers oder ein Marker-Teilelement von einer Kamera, welche im Wesentlichen nicht mit einer Bewegung des Instruments mitgeführt wird, erfasst werden kann. Eine solche Markeranordnung erleichtert auch die Sterilisierung, da glatte Oberflächen einfacher und leichter zu desinfizieren sind, als an einem Lager auf einem Referenzstern angebrachte Marker.

Im Allgemeinen ist ein Marker bzw. ein Marker-Teilelement ein Element, welches Licht emittieren kann. Dies kann zum Beispiel aktiv erfolgen, indem LEDs oder andere lichtemittierende Elemente verwendet werden, oder es können reflektierende Oberflächen verwendet werden, welche Licht, welches von außen auf den Marker auftrifft, reflektieren können. Dabei kann es sich zum Beispiel um sichtbares Licht oder um Infrarotstrahlen handeln, welche anschließend von mindestens einer Kamera erfasst werden.

Der Marker ist bevorzugt ein in etwa rotationssymmetrisches Element, zum Beispiel ein ringförmiges oder bandförmiges Element, welches auf ein Instrument, bevorzugt ein in etwa rotationssymmetrisches Instrument entlang der Längsachse aufgeschoben und bevorzugt fixiert werden können oder welche zum Beispiel von seitlich auf das Instrument aufgeclippt oder verrastet, oder zum Beispiel als Klebeband mit reflektierender Oberfläche umlaufend auf dem Instrument angeordnet werden kann.

Weiterhin ist es möglich, den Marker zum Beispiel kugelförmig oder kegelförmig auszugestalten, wobei das kugelförmige oder kegelförmige Element mit reflektierender Oberfläche so um das Instrument herum angeordnet werden kann, dass bei einer Verschiebung oder Drehung des Instruments mindestens ein Teilbereich der Oberfläche von einer seitlich zum Instrument angeordneten Kamera erfasst werden kann.

Allgemein ist es jedoch nicht erforderlich, dass der Marker, ein Marker-Teilelement oder das Instrument, an welchem der Marker oder ein Marker-Teilelement angebracht wird, rotationssymmetrisch ist. Der Marker kann zum Beispiel auch nicht-rotationssymmetrisch ausgestaltet sein, d.h. es ist im Sinne der Erfindung ausreichend, wenn mindestens ein Teilbereich eines Markers bei bevorzugt jeder räumlichen Ausrichtung oder Verdrehung des Instruments, an welchem der Marker angebracht ist, erfasst werden kann. Werden nicht-rotationssymmetrische Marker oder Marker-Teilelemente verwendet, so ist es möglich, bei geeigneter Anordnung von verschiedenen Marker-Abmessungen um das Instrument herum anhand der sich bei verschiedenen Drehungen oder Ausrichtungen des Instruments erfassbaren Markerflächen festzustellen, wie das Instrument gerade gedreht oder räumlich ausgerichtet ist.

Vorteilhaft werden mindestens zwei Marker oder Marker-Teilelemente an einem Instrument vorgesehen, um die Präzision der räumlichen Erfassung über von den Markern ausgesandtes oder reflektiertes Licht zu erhöhen. Dabei sind die mindestens zwei Marker vorteilhaft mit einem bestimmten Abstand dazwischen versetzt zueinander angeordnet, zum Beispiel bei einem rotationssymmetrischen Instrument axialer Richtung bzw. in Richtung der Symmetrieachse voneinander beabstandet.

Nach einem Aspekt der Erfindung wird ein Verfahren zur Lokalisation nach Anspruch 1 vorgeschlagen.

Wird das von einem Marker ausgesandte oder reflektierte Licht von einer Kamera erfasst, welche nicht auf einer Linie senkrecht zu der Oberfläche des Markers angeordnet ist, so entsteht aufgrund der schrägen Betrachtungsweise ein Fehler bezüglich der erfassten räumlichen Lage, welcher erfindungsgemäß korrigiert wird, um aus den erfassten Daten die richtige räumliche Lage zu ermitteln. Dabei wird vorteilhaft ein iteratives Verfahren eingesetzt, wobei zunächst davon ausgegangen wird, dass die ermittelte Position des mindestens einen Markers, zum Beispiel das Reflexionszentrum eines ringförmigen Markers, die korrekte Position des Ringes darstellt, welche jedoch aufgrund einer möglicherweise schrägen Betrachtung von der tatsächlichen Position abweicht, da bei einer Erfassung des Markers unter einem schrägen Winkel der von einer Kamera erfasste Mittelpunkt der reflektierenden Fläche von dem tatsächlichen Mittelpunkt des Markers abweicht. Ausgehend von der vorläufigen Annahme, dass die erfasste Position die korrekte Position des Markers bzw. Ringes ist, wird die Sichtlinie der mindestens einen, bevorzugt zwei oder mehrerer Erfassungskameras für jeden Marker bzw. Ring bestimmt. Aus diesen Sichtlinien wird ein Korrekturwert für jede Kamera und/oder jeden Marker bzw. Ring berechnet und daraus werden virtuelle Positionen eines Markers bzw. Ringes als Mittelpunkt zwischen den Sichtlinien der Kameras bestimmt. Hieraus kann ein Korrekturvektor oder eine Verschiebung zwischen der als korrekt angenommenen Position und der wie oben bestimmten virtuellen Position ermittelt werden. Anschließend wird von der ursprünglich als richtig angenommenen Position, welche von der tatsächlich richtigen Position abweicht, der Korrekturvektor subtrahiert, um einen besseren oder bestenfalls sogar richtigen Wert für die richtige Position zu erhalten. Ausgehend von diesem korrigierten Wert der als richtig angenommenen Position wird das Verfahren iterativ wiederholt, bis die Abweichung oder der Korrekturvektor einen bestimmten vorgebbaren Grenzwert unterschreiten, d.h. bis eine vorgegebene Genauigkeit erreicht ist.

Eine weitere, nicht zur Erfindung gehörende, Ausführungsform bezieht sich auf ein Verfahren zum Bestimmen der räumlichen Lage mindestens eines Markers, wobei die räumliche Lage des mindestens einen Markers iterativ bestimmt wird. Das oben in Grundzügen dargestellte iterative Verfahren kann allgemein auch bei beliebig ausgestalteten Markern oder Markeranordnungen verwendet werden, um die räumliche Lage mindestens eines Markers, wie zum Beispiel drei auf einem Referenzstem angeordneten Markern zu bestimmen. Dabei wird in einem ersten Schritt das von dem mindestens einen Marker, bevorzugt zwei, drei oder mehr Markern, ausgesandte, zum Beispiel aktiv emittierte oder passiv reflektierte Licht beispielsweise mit einer, zwei oder mehr Kameras optisch erfasst, wobei hieraus unter Kenntnis der relativen Anordnung der einzelnen Marker zueinander, zum Beispiel der Geometrie des Referenzsterns, die räumliche Lage der Markeranordnung ungefähr bzw. approximativ bestimmt werden kann. Ist die räumliche Lage einer Markeranordnung bekannt, so kann hieraus auch die räumliche Lage eines mit der Markeranordnung verbundenen Instruments ermittelt werden. Wird zum Beispiel zunächst davon ausgegangen, dass die einzelnen Marker kugelförmig bzw. kugelsymmetrisch oder kugelförmige reflektierende Oberflächen sind, so wird der Mittelpunkt des von jedem Marker ausgesandten Lichtes vorläufig als Markermittelpunkt oder Position des Markers angenommen. Werden nicht-kugelförmige Marker verwendet, so ist bei einer vorgegebenen Anordnung der einzelnen Marker zum Beispiel auf einem Referenzstern die Orientierung dieser Marker auf dem Referenzstem bereits vor Durchführung des Verfahrens bekannt. Unter Verwendung dieser Information in Verbindung mit der approximativ bestimmten räumlichen Lage der Markeranordnung, zum Beispiel des Referenzsterns, sowie unter Berücksichtigung der bekannten Markergeometrie - also zum Beispiel der Information, dass beispielsweise kegelförmige oder zylinderförmige Marker verwendet werden - sowie unter Berücksichtigung der bekannten Position mindestens einer zur optischen Erfassung dienenden Kamera und gegebenenfalls der Position mindestens eines lichterzeugenden Elementes, falls passive reflektierende Marker verwendet werden, kann eine Simulation durchgeführt werden, um zu ermitteln, welche optischen Signale bei der approximativ ermittelten räumlichen Lage der Markeranordnung hätten empfangen werden sollen. Stimmen die in der Simulation ermittelten empfangenen optischen Signale mit den tatsächlich empfangenen optischen Signalen überein, so ist die approximativ ermittelte räumliche Lage der Markeranordnung korrekt. Tritt jedoch eine Abweichung oder Differenz zwischen simulierten zu empfangenden optischen Signalen und tatsächlich empfangenen optischen Signalen auf, so kann hieraus eine Korrekturgröße, wie zum Beispiel eine Differenz oder ein dreidimensionaler Korrekturvektor oder Verschiebungsvektor ermittelt werden, um welchen zum Beispiel ein Marker verschoben oder die Markeranordnung gedreht werden muss, um eine bessere Approximation oder sogar die korrekte räumliche Lage der Markeranordnung zu erhalten. Nach der Korrektur oder Verschiebung der mindestens einen approximativ bestimmten räumlichen Position der Markeranordnung kann eine erneute Simulation der zu empfangenden optischen Signale unter Verwendung der oben erwähnten Informationen durchgeführt werden, um zu ermitteln, ob die nach der erneuten Simulation errechneten zu empfangenden optischen Signale besser oder vollständig mit den tatsächlich empfangenen optischen Signalen übereinstimmen. Hierbei kann wiederum ein Korrekturwert bzw. eine Differenz zwischen tatsächlich empfangenen optischen Signalen und simulierten zu empfangenden optischen Signalen ermittelt werden, wobei der Korrekturwert wiederum für eine weitere Simulation verwendet werden kann. Dieses Verfahren kann iterativ zum Beispiel solange fortgesetzt werden, bis ein vorgegebener zulässiger Toleranzwert für einen Fehler unterschritten oder sogar die vollständige Übereinstimmung zwischen simulierten und tatsächlich empfangenen optischen Signalen erreicht wird. Es kann zum Beispiel als Abbruchbedingung auch die Anzahl der maximal durchzuführenden Iterationsschritte vorgegeben werden.

Zur Durchführung des Verfahrens können vorteilhaft Marker verwendet werden, welche keine kugelförmigen Oberflächen aufweisen, wie zum Beispiel zweidimensionale oder dreidimensionale Gebilde, zum Beispiel zylinderförmige, kegelförmige oder würfelförmige Objekte. Dies vereinfacht die Herstellung und Sterilisierung der Marker, da es zum Beispiel relativ aufwändig ist auf kugelförmige Oberflächen reflektierende Beschichtungen oder Folien aufzubringen und diese weiterhin relativ aufwändig zu desinfizieren sind, im Gegensatz zu zum Beispiel kegelförmigen oder zylinderförmigen Objekten. Allgemein können die Marker aktive lichtemittierende Elemente, wie zum Beispiel LEDs, oder auch passive reflektierende Elemente oder Oberflächen sein.

Die Erfindung bezieht sich gemäß einem weiteren Aspekt auf ein Computerprogramm, welches das oben beschriebene erfindungsgemäße Verfahren durchführt, wenn es in einem Computer geladen ist bzw. wird oder auf einem Computer läuft. Weiterhin bezieht sich die Erfindung auch auf ein Programmspeichermedium oder ein Computerprogrammprodukt mit dem oben erwähnten Programm.

Nach einem weiteren Aspekt bezieht sich die Erfindung auf eine Vorrichtung zum Bestimmen der räumlichen Lage mindestens eines Markers mit einer Kamera und einer Recheneinheit zur Durchführung des oben beschriebenen erfindungsgemäßen Verfahrensschrittes. Dabei können lichtemittierende Elemente entweder als Marker selbst oder getrennt von den Markern, zum Beispiel in einem bekannten Lageverhältnis zu der mindestens einen Kamera oder auch um eine einzelne Kamera herum als etwa ringförmiges Element vorgesehen sein.

Weiterhin bezieht sich die Erfindung auf ein System mit einer oben beschriebenen Vorrichtung und mindestens einem Marker, bevorzugt einer Markeranordnung aus zwei, drei oder mehr Markern, welche von der mindestens einen Kamera erfasst werden können. Dabei haben die verwendeten Marker bevorzugt eine zweidimensionale oder dreidimensionale Struktur, wobei vorteilhaft keine kugelförmigen Oberflächen vorhanden sind. So können beispielsweise zylinderförmige, kegelförmige oder würfelförmige Marker oder auch Marker mit einer anderen Geometrie, wie zum Beispiel kegelstumpfförmige oder ellipsenförmige Marker verwendet werden.

Die Erfindung wird nachfolgend anhand eines bevorzugten Ausführungsbeispieles eines ringförmigen Markers unter Bezugnahme auf die beiliegenden Figuren beschrieben werden. Es zeigen:

Figur 1     eine Ausführungsform eines Markers auf einem zylinderförmigen Instrument;
Figur 2     eine perspektivische Ansicht eines Markers;
Figur 3     ein Schnittbild des Markers in der y-z-Ebene; und
Figur 4     ein Schnittbild des Markers in der x-y-Ebene.

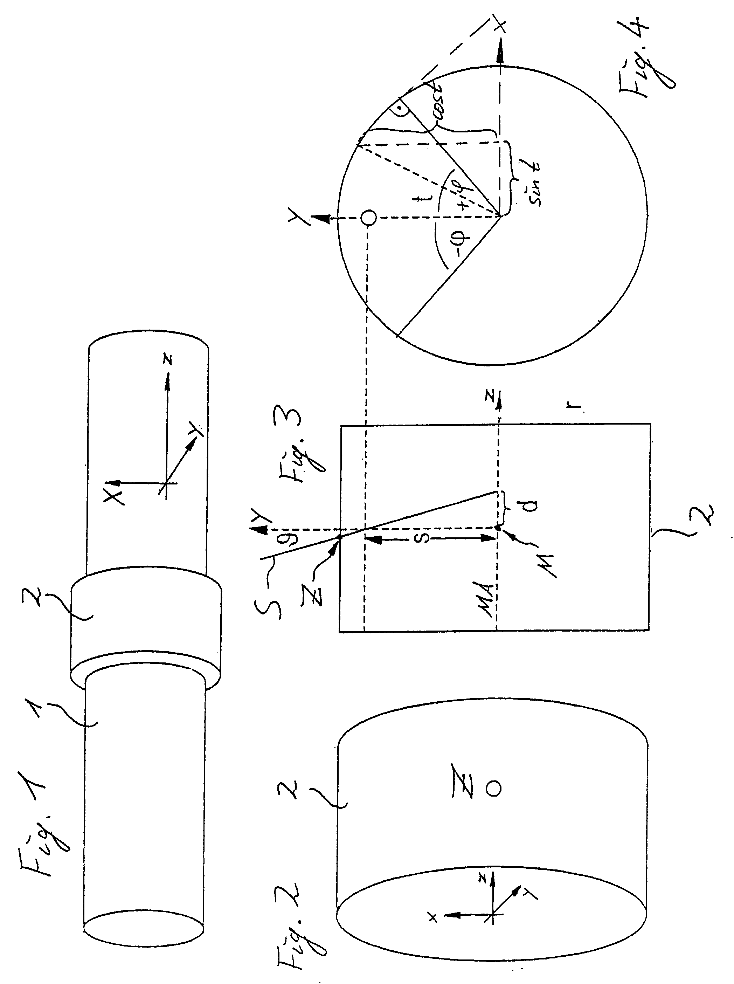

Figur 1 zeigt ein zylinderförmiges Instrument 1, wie zum Beispiel die Achse eines Bohrers oder Schraubenziehers, welche beispielsweise für medizinische Zwecke eingesetzt werden können. Auf dem zylinderförmigen Instrument 1 ist ein ringförmiger Marker 2 angebracht, so dass unabhängig von einer Verdrehung des zylinderförmigen Elements 1 immer eine Oberfläche des Markers 2 von einer seitlich zu dem zylinderförmigen Element 1 angeordneten Kamera erfasst werden kann. Zur Erläuterung der nachfolgenden Figuren ist in Figur 1 ein x-y-z-Koordinatensystem eingezeichnet.

Figur 2 zeigt eine perspektivische Ansicht des ringförmigen Markers 2, so wie der Marker 2 von einer Kamera (nicht gezeigt) erfasst wird, welche nicht auf einer Linie senkrecht auf der Markeroberfläche angeordnet ist, sondern welche schräg auf den Marker 2 blickt. Das in Figur 2 eingezeichnete Reflexionszentrum Z ist der Mittelpunkt der von der Kamera aus betrachteten sichtbaren Fläche des Markers 2, welcher jedoch aufgrund der schrägen Betrachtung von dem richtigen Mittelpunkt des Markers 2 abweicht, welcher in dem in Figur 2 gezeigten Beispiel rechts neben dem Reflexionsmittelpunkt Z liegt.

Figur 3 ist ein Schnitt durch den in Figur 2 gezeigten Marker 2 und stellt die y-z-Ebene dar. Die schräg auf den Marker 2 blickende Kamera erfasst den Marker 2 aus Richtung der Sichtlinie S, welche durch das Reflexionszentrum Z hindurchgeht und die Mittelachse MA des ringförmigen Markers 2 im Abstand d vom Mittelpunkt M der Mittelachse MA schneidet. Würde man das von der Kamera erfasste Reflexionszentrum Z als wahren Mittelpunkt des Markers 2 verwenden und dabei die Ungenauigkeit aufgrund der schrägen Betrachtung des Markers 2 außer Acht lassen, so würde man den Positionsfehler d bezüglich der räumlichen Lage des Markers 2 und damit des mit dem Marker 2 verbundenen Instruments erhalten. Um diesen Positionsfehler d zu korrigieren, wird zunächst davon ausgegangen, dass das bestimmte Reflexionszentrum Z in der Mitte der Markeroberfläche liegt. Werden auf dem Instrument 1 zwei Marker 2 mit einem bekannten Abstand voneinander angebracht, so kann, basierend auf der Annahme, dass die für die beiden Marker 2 bestimmten Reflexionszentren Z1 und Z2 (nicht gezeigt) die richtigen Reflexionszentren sind, die Sichtlinie S und damit der Winkel $\vartheta$ ermittelt werden, unter welchem die Sichtlinie S zur Senkrechten auf die Oberfläche des Markers 2 gezeigt ist.

Figur 4 zeigt einen Schnitt durch den Marker 2 in der x-y-Ebene. Wird mit t der Winkel zur y-Achse bezeichnet, so ist der Normalenvektor auf die Oberfläche des Markers 2 gegeben durch (sin t; cos t; 0). Der Richtungsvektor der Sichtlinie S, wie in Figur 3 gezeigt, ist gegeben durch (0; cos $\vartheta$; sin $\vartheta$). Der Winkel $\beta$ zwischen der Normalen auf die Oberfläche und der Sichtlinie S ist gegeben durch:

$$\cos t \cdot \cos \vartheta = \cos \beta$$

Die lokale Fläche, welche von einer Kamera aus Richtung der Sichtlinie S gesehen werden kann, ist proportional zu cos $\beta$. Dieser Winkel $\beta$ darf den kritischen Reflexionswinkel $\alpha$ (nicht gezeigt) des reflektierenden Filmes nicht überschreiten, welcher zum Beispiel ungefähr 50° beträgt. Demzufolge ist der maximale Wert $\varphi$, den t betragsmäßig annehmen kann, definiert durch

$$\cos \varphi = \cos \alpha / \cos \vartheta$$

Insgesamt kann von einer Kamera, welche in Figur 4 oberhalb des reflektierenden Ringes 2 angeordnet ist, ein Oberflächenbereich beginnend vom Winkel -$\varphi$ bis zum Winkel +$\varphi$ erfasst werden. Um die in Figur 3 gezeigte Verschiebung d zu bestimmen, um welche das Reflexionszentrum Z von dem wahren Mittelpunkt M der Mittelachse MA des reflektierenden Körpers 2 verschoben ist, wird die in Figur 3 eingezeichnete Strecke s bestimmt. Wie in Verbindung mit Figur 4 gesehen wird, ist die Strecke s der Schwerpunkt des von einer Kamera erfassbaren Kreisbogens im Winkelbereich -$\varphi$ bis +$\varphi$. s kann berechnet werden aus:

$$s= \int_{-\varphi}^{\varphi} r \cdot \cos t \cdot \cos t \cdot \cos \vartheta \cdot r \cdot dt \quad\Bigg/\quad \int_{-\varphi}^{\varphi} \cos t \cdot \cos \vartheta \cdot r \cdot dt = \frac{r}{2} \left[ \frac{\varphi}{\sin\varphi} + \cos\varphi \right]$$

Damit ergibt sich die Verschiebung bzw. der Korrekturwert d durch:

$$d = s \cdot \tan\vartheta$$

Dieses Verfahren kann iterativ durchgeführt werden.-Somit kann die richtige Position des reflektierenden Ringes 2 auf dem zylinderförmigen Körper 1 aus der von einer Kamera erfassten Position des Reflexionszentrums Z berechnet werden und damit kann die Position des zylinderförmigen Körpers 1 im Raum bestimmt werden.

**Patentansprüche**

1. Verfahren zum Lokalisieren mindestens eines Instruments mit mindestens zwei ringförmigen Markern (2), welche entlang Teilbereichen einer um das Instrument (1) umlaufenden Oberfläche angeordnet werden können, wobei die Position des Reflexionszentrums (Z) der mindestens zwei Marker (2) erfasst wird und hieraus ein Korrekturwert (d) zur Bestimmung der räumlichen Position des wahren Mittelpunkts (M) jedes der mindestens zwei Markern (2) und somit des mit diesen verbundenen Instruments ermittelt wird.

2. Verfahren nach Anspruch 1, **dadurch gekennzeichnet, dass** die Bestimmung des Korrekturwerts (d) der räumlichen Lage des Instruments (1) iterativ durchgeführt wird.

3. Verfahren nach Anspruch 2, wobei unter Verwendung einer vorher bekannten relativen Lageinformation einer Anordnung von mindestens zwei Marken (2) die räumliche Lage der Markeranordnung aus den optisch erfassten Lichtsignalen approximativ bestimmt wird.

4. Verfahren nach Anspruch 3, wobei unter Verwendung der approximativ bestimmten räumlichen Lage der Markeranordnung, der vorher bekannten Orientierung der einzelnen Markerelemente (2) auf der Markeranordnung und der Position der optischen Erfassungsvorrichtung eine Simulation von zu empfangenden optischen Signalen durchgeführt wird.

5. Verfahren nach Anspruch 4, wobei die simulierten zu empfangenden optischen Signale mit den tatsächlich empfangenen optischen Signalen verglichen werden und hieraus eine Korrekturgröße ermittelt wird.

6. Verfahren nach Anspruch 5, wobei die approximativ bestimmte räumliche Lage der Markeranordnung unter Verwendung der ermittelten Korrekturgröße korrigiert wird, um einen neuen Wert der approximativ bestimmten räumlichen Lage der Markeranordnung zu erhalten und eine weitere Simulation nach Anspruch 4 durchgeführt wird.

7. Verfahren nach Anspruch 6, wobei eine Abbruchbedingung für das iterative Verfahren vorgegeben wird.

8. Computerprogramm, welches das Verfahren nach einem der Ansprüche 1 bis 7 durchführt, wenn es in einen Computer geladen ist oder auf einem Computer läuft.

9. Programmspeichermedium oder Computerprogrammprodukt mit dem Computerprogramm nach Anspruch 8.

10. Vorrichtung zum Erkennen der räumlichen Lage mindestens zweier Marker ringförmiger (2) mit mindestens einer Kamera und einer Recheneinheit zur Durchführung des Verfahrens nach einem der Ansprüche 1 bis 7.

11. Vorrichtung nach Anspruch 10, wobei mindestens ein lichtemittierendes Element in einem definierten Lageverhältnis zu der mindestens einen Kamera vorgesehen ist.

**12.** System mit einer Vorrichtung nach einem der Ansprüche 10 oder 11 und einer Anordnung von mindestens zwei Markern.

**13.** System nach Anspruch 12, wobei die Marker nicht-kugelsymmetrisch ausgebildet sind.

**Claims**

**1.** A method for localising at least one instrument comprising at least two ring-shaped markers (2) which can be arranged along partial areas of a surface encircling said instrument (1), wherein the position of the reflection centre (Z) of the at least two markers (2) is detected and from this, a correction value (d) for determining the spatial position of the true centre point (M) of each of the at least two markers (2) and thus of the instrument (1) connected to said at least two markers (2) is determined.

**2.** The method as set forth in claim 1, **characterised in that** said correction value (d) for the spatial position of said instrument (1) is iteratively determined.

**3.** The method as set forth in claim 2, wherein the spatial position of an arrangement of at least two markers (2) is approximately determined from said optically detected light signals, using information known beforehand about the relative position of said arrangement of markers.

**4.** The method as set forth in claim 3, wherein a simulation of the optical signals to be received is carried out, using said approximately determined spatial position of said arrangement of markers, said orientation of the individual marker elements (2) on said arrangement of markers known beforehand and the position of the optical detection device.

**5.** The method as set forth in claim 4, wherein said simulated optical signals to be received are compared with the optical signals actually received, from which a correction variable is determined.

**6.** The method as set forth in claim 5, wherein said approximately determined spatial position of said arrangement of markers is corrected using said correction variable determined, in order to obtain a new value for said approximately determined spatial position of said arrangement of markers, and another simulation as set forth in claim 4 is carried out.

**7.** The method as set forth in claim 6, wherein a termination condition is predetermined for said iterative method.

**8.** A computer program which performs the method as set forth in any one of claims 1 to 7 when it is loaded onto a computer or is running on a computer.

**9.** A program storage medium or computer program product comprising said computer program as set forth in claim 8.

**10.** A device for identifying the spatial position of at least two ring-shaped markers (2), comprising at least one camera and a computational unit for performing the method as set forth in any one of claims 1 to 7.

**11.** The device as set forth in claim 10, wherein at least one light-emitting element is provided in a defined positional relationship with respect to the at least one camera.

**12.** A system comprising a device as set forth in any one of claims 10 or 11 and an arrangement of at least two markers.

**13.** The system as set forth in claim 12, wherein said markers are formed non-spherical-symmetrically.

**Revendications**

**1.** Procédé de localisation d'au moins un instrument avec au moins deux marqueurs annulaires (2), qui peuvent être disposés le long de zones partielles d'une surface entourant l'instrument (1), la position du centre de réflexion (Z) des au moins deux marqueurs (2) étant saisie et une valeur de correction (d) de détermination de la position spatiale du point central vrai (M) de chacun des au moins deux marqueurs (2) et donc de l'instrument relié à ceux-ci étant déterminée à partir de là.

2. Procédé suivant la revendication 1, **caractérisé en ce que** la détermination de la valeur de correction (d) de la position spatiale de l'instrument (1) est réalisée de manière itérative.

3. Procédé suivant la revendication 2, dans lequel, moyennant utilisation d'une information de position relative préalablement connue d'une disposition d'au moins deux marqueurs (2), la position spatiale de la disposition de marqueurs est déterminée approximativement à partir des signaux lumineux saisis par voie optique.

4. Procédé suivant la revendication 3, dans lequel, sur base de la position spatiale déterminée approximativement de la disposition de marqueurs, de l'orientation préalablement connue des éléments de marqueurs individuels (2) sur la disposition de marqueurs et de la position du dispositif de saisie optique, on effectue une simulation des signaux optiques à recevoir.

5. Procédé suivant la revendication 4, les signaux optiques à recevoir simulés étant comparés avec les signaux optiques réellement reçus et une grandeur de correction étant déterminée à partir de là.

6. Procédé suivant la revendication 5, la position spatiale déterminée approximativement de la disposition de marqueurs étant corrigée avec utilisation de la grandeur de correction déterminée afin d'obtenir une nouvelle valeur de la position spatiale déterminée approximativement de la disposition de marqueurs et une nouvelle simulation étant effectuée suivant la revendication 4.

7. Procédé suivant la revendication 6, une condition d'interruption étant prédéfinie pour le procédé itératif.

8. Programme d'ordinateur qui effectue le procédé suivant l'une des revendications 1 à 7 lorsqu'il est chargé dans un ordinateur ou tourne sur un ordinateur.

9. Support d'information pour programme ou produit de programme d'ordinateur comportant le programme d'ordinateur suivant la revendication 8.

10. Dispositif de détection de la position spatiale d'au moins deux marqueurs annulaires (2) avec au moins une caméra et une unité de calcul pour la réalisation du procédé suivant l'une des revendications 1 à 7.

11. Dispositif suivant la revendication 10, au moins un élément émetteur de lumière étant prévu dans une position relative définie par rapport à la au moins une caméra.

12. Système avec un dispositif suivant l'une des revendications 10 ou 11 et une disposition d'au moins deux marqueurs.

13. Système suivant la revendication 12, les marqueurs n'étant pas de forme à symétrie sphérique.

Fig. 1

Fig. 2

Fig. 3

Fig. 4